# EUROPÄISCHE PATENTSCHRIFT

(11) **EP 1 021 191 B1**
(45) Veröffentlichungstag und Bekanntmachung des Hinweises auf die Patenterteilung: **05.06.2002**
(21) Anmeldenummer: 98950078.0
(22) Anmeldetag: 24.09.1998
(51) Int. Cl.: A61K 31/565

(54) **STRAFFUNG UND/ODER VERKLEINERUNG VON FETTZELLEN-HALTIGEN KÖRPERPARTIEN**
TIGHTENING AND/OR REDUCING THE SIZE OF BODY PARTS CONTAINING FAT CELLS
RAFFERMISSEMENT ET/OU REDUCTION DE LA TAILLE DE PARTIES DU CORPS RENFERMANT DES CELLULES ADIPEUSES

(30) Priorität: 08.10.1997 DE 19744451
(43) Veröffentlichungstag der Anmeldung: 26.07.2000
(73) Patentinhaber: Genesiscosmetics Rights & Licenses Company Limited, 9490 Vaduz (LI)
(72) Erfinder: SCHMIDT, Alfred, D-22301 Hamburg (DE); WIELAND, Heinrich, D-79271 St. Peter (DE)
(74) Vertreter: Leson, Thomas Johannes Alois, Dipl.-Ing.
(86) Internationale Anmeldenummer: EP9806085
(87) Internationale Veröffentlichungsnummer: WO9917712

(56) Entgegenhaltungen:
- WO-A-97/36570
- US-A- 4 895 715
- US-A- 4 937 250
- BRODIE A.M.H.: "Aromatase inhibition and its pharmacologic inplications." BIOCHEMICAL PHARMACOLOGY, (1985) 34/18 (3213-3219). CODEN: BCPCA6, XP002101635 United Kingdom

## Beschreibung

Die vorliegende Erfindung betrifft die Verwendung einer Substanz oder einer diese Substanz enthaltenden Zusammensetzung, die zur Straffung und/oder Verkleinerung von Fettzellen-haltigen Körperpartien besonders gut geeignet ist.

Die vorliegende Erfindung ist verwandt mit der nachveröffentlichten, älteren Patentanmeldung WO-A-97/36570 insofern, als dort gleichartige Substanzen bzw. Zusammensetzungen offenbart sind. Ein Unterschied besteht jedoch darin, daß es im vorgenannten Dokument um Applikationen bei gestörtem Unterhaut-Bindefettgewebe geht, während die vorliegende Erfindung die Straffung und/oder Verkleinerung von Fettzellen-haltigen Körperpartien zum Ziel hat.

Die Ausbildung der typischen Verteilung des Körperfettes dient theleologisch gesehen in besonderem Maße der Arterhaltung des Menschen. Neben dem zweifellos vorhandenen psychologischen und ästhetischen Effekt steht der biologische Effekt im Vordergrund, da diese Verteilung sowohl ausreichend Kalorien speichert - um beispielsweise bei der weiblichen Brust eine Schwangerschaft plus Ernährung des Säuglings zu gewährleisten - als auch die entsprechenden Körperteile formt und zur Bewerkstelligung der Ernährung mit Kalorien (in Form von Fettgewebe) versieht.

Allerdings kann sich diese an sich normale und typische Verteilung des Fettgewebes, sei es physiologisch bedingt oder aufgrund falscher Ernährung, leicht verändern, und es kann sich - insbesondere in fortgeschrittenem Alter - eine nicht erwünschte, anormale Verteilung des Körperfettes herausbilden. Die weibliche Brust kann - häufig durch eine Schwangerschaft ausgelöst - mit zunehmendem Alter der Frau ein Erschlaffen oder ein unerwünschtes Vergrößern erfahren. Das Erscheinungsbild solcher erschlaffter oder mit anormaler Körperfettverteilung gekennzeichneter Körperpartien kann zu einer erheblichen psychologischen Belastung führen. Nicht selten wünschen sich Frauen mit erschlaffter oder zu groß gewachsener Brust eine Straffung oder Verkleinerung der Brust.

Das Bedürfnis zur Straffung und/oder Verkleinerung von Fettzellen-haltigen Körperpartien konnte bisher in der Praxis wirksam nur durch eine operative Behandlung befriedigt werden. Ein solche operative Behandlung ist jedoch mit erheblichen Nachteilen verbunden. Die Operation erfordert einen naturgemäß relativ aufwendigen chirurgischen Eingriff. Ferner bleiben operationsbedingt Narben zurück, die häufig nicht zu verbergen sind und somit wiederum zu einer psychologischen Beeinträchtigung führen. Je nachdem, welcher Körperbereich betroffen ist, kann es sein, daß die operativen Maßnahmen oder die Vernähung der zurückbleibenden Hautpartien nach der operativen Entfernung von Fettgewebe schwierig sind.

Aufgabe der vorliegenden Erfindung ist es daher, die Behandlungsmöglichkeiten zur Straffung und/oder Verkleinerung von Fettzellen-haltigen Körperpartien unter Beseitigung der vorstehend genannten Nachteile zu verbessern.

Die Aufgabe wird gelöst durch die Verwendung einer Substanz, die die Bildung und/oder die Wirkung von Östrogenen hemmt, bei lokal topischer Applikation zur Straffung und/oder Verkleinerung von Fettzellen-haltigen Körperpartien. Alternativ und bevorzugt liegt die Substanz in einer lokal topisch zu applizierenden Zusammensetzung enthaltend vor.

Ein weiterer Gegenstand der Erfindung besteht in einer Verwendung der vorstehend bezeichneten Substanz oder Zusammensetzung zur Herstellung eines Mittels zur Straffung und/oder Verkleinerung von Fettzellen-haltigen Körperpartien. Das Mittel wird dabei in erster Linie für kosmetische Zwecke eingesetzt.

Bevorzugte Ausführungsformen der Erfindung sind in den Unteransprüchen zu Anspruch 1 gekennzeichnet. Die Erfindung ist auf besonders geeignete Weise einsetzbar zur Straffung und/oder Verkleinerung des Brustbereichs, insbesondere der weiblichen Brust.

Die vorliegende, erfindungsgemäße Verwendung ermöglicht einen neuartigen Behandlungsweg. Sie geht nicht einen herkömmlichen Behandlungsweg, der über operative/chirurgische Mäßnahmen oder durch systemische Medikationssysteme vollzogen wird. Vielmehr haben die Erfinder überraschend festgestellt, daß der Einsatz der speziellen Substanz als Wirksubstanz eine lokal topische Applikation auf die Haut zuläßt, die zu einer besonders wirksamen Straffung und/oder Verkleinerung von Fettzellen-haltigen Körperpartien führt.
Danach greift die erfindungsgemäß einzusetzende Substanz, die eine Hemmung auf die Bildung und/der die Wirkung von Östrogen ausübt, an Ort und Stelle in die extragonadale Östrogenbildung und die lokale Wirkung dieses Hormontyps ein, welcher ansonsten die Umwandlung von Bindegewebszellen in Fettzellen verursacht. Die resultierende Verminderung der lokalen Östrogenkonzentration gemäß der Erfindung führt zu einer gegenläufigen Entwicklung; die Bindegewebszellen werden in geringerem Maße oder nicht mehr in Fettzellen umgewandelt, und die Fettzellen vermindern ihren Füllungsgrad. Gleichzeitig kann eine günstige Umstrukturierung und Straffung der Bindegewebsstruktur erzielt werden.
Ferner wird die Hautoberfläche im Laufe der lokal topischen Anwendung der die Bildung und/oder die Wirkung von Östrogenen hemmenden Substanz in den behandelten Fettzellen-haltigen Korperpartien zunehmend glatt und gestrafft.
Ein besonderer Vorteil des erfindungsgemäß ermöglichten Behandlungsweges, insbesondere gegenüber einer grundsätzlich in Betracht zu ziehenden systemischen Behandlung über die Blutbahn, ergibt sich daraus, daß die Straffung und/oder Verkleinerung der Fettkörper-haltigen Körperteile sehr gezielt und selektiv erreicht werden kann, ohne jedoch das physiologische Gleichgewicht mit dem normalen Östrogen-Körperhaushalt im unbehandelten, also weitaus größten Körperbereich wesentlich zu verändern.

So wird gemäß der vorliegenden Erfindung durch die lokale Anflutung der topisch eingesetzten Wirksubstanz faktisch nur die Östrogenbildung bzw. -wirkung im peripheren, behandelten Körperbereich gehemmt. Eine androgene Wirkung ist nicht zu erwarten und konnte auch nicht nachgewiesen werden. Die erfindungsgemäß eingesetzten Substanzen wirken nur lokal, also nicht systemisch. Bei den behandelten Frauen traten keinerlei Unverträglichkeiten auf.
Die ohne Umweg über den Blutkreislauf direkt zu dem Wirkort gelangenden und lokal hemmenden Wirksubstanz bewirkt eine deutliche Verkleinerung der Fettzellen, verbunden mit einer Zunahme der Bindegewebsfasern, was im Endeffekt zu einer Reduzierung und Straffung des Fettgewebes führt.
Der neue Wirk- und Behandlungsmechanismus wurde an verschiedenen Probandinnen, die ihre weibliche Brust erfindungsgemäß behandelten, bestätigt. Es ließen sich ohne weiteres Reduktionen um bis zu 30 % erreichen. Damit könnte eine in vielen Fällen induzierte operative Brustverkleinerung vermieden oder vorbereitet werden.

Der Wirkungseintritt und somit die Straffung und/oder Verkleinerung findet bei ständiger lokaler und topischer Applikation (beispielsweise 1-2mal täglich) auf die Fettzellen-haltigen Körperpartien bereits innerhalb weniger Wochen statt.

Unter dem Begriff "Östrogene" sind alle natürliche, weibliche Sexualhormone mit östrogener Wirkung zu verstehen, wie Östradiol, Östron und Östrol.
Als im Hinblick auf Östrogene bildungs- und/oder wirkungshemmende Substanzen kommen gemäß der vorliegenden Erfindung insbesondere zwei Klassen von Substanzen in Betracht, die im Folgenden näher beschrieben werden.

Einmal sind dies Anti-Östrogene, d.h.. solche Substanzen, die Östrogenrezeptoren blockieren und somit als Antagonisten die Wirkung von Östrogen hemmen.

Ferner sind dies Substanzen, die lokal die extragonadale Östrogen-Bildung hemmen können. Hierfür kommen vor allem steroidale und nicht-steroidale Inhibitoren der (Cytochromp450)-Aromatase in Frage. Diese Aromatase stellt das zentrale Enzym dar, welches in den Stromazellen des Fettgewebes die chemische Umwandlung der aus der Nebenniere stammenden und über das Blut transportierten Vorläufer-Moleküle (wie Dehydroantrosteron, DHEA und Androstendion) in Östrogene katalysiert. Die Hemmung dieses Enzyms führt folglich zu einer lokal-topischen *in situ*-Hemmung der Östrogen-Bildung. Aufgrund ihrer besonders vorteilhaften Wirkungsweise sind die Aromatase-Inhibitoren zum Einsatz bei der erfindungsgemäßen Verwendung bevorzugt.

Beispiele für Aromatase-Inhibitoren sind die Substanzen 4-Hydroxyandrost-4-en-3,17-dion (Formestan™), 6-Methylenandrostra-1,4-dien-3,17-dion (Exemestan™), 10-(2-Propynyl)estr-4-en-3,17-dion (MDL 18962) und 7α-substituierte Androstendion-Derivate als Beispiele für steroidale Aromataseinhibitoren sowie Imidazol- und Triazol-Derivate als Beispiele für nicht-steroidale Aromataseinhibitoren, wie 6-[(4-Chlorophenyl) (1H-1,2,4-triazol-1-yl)-methyl] 1-methyl-1H-benzotriazol (Vorazol™), 2,2'-[5-(1H-1,2,4-triazol-1-yl methyl)-1,3-phenylen]bis(2-methylproprionitril) (Arimidex™), 4-[1-(Cyanophenyl)-1-(1,2,4-triazolyl)methyl]benzonitril (Letrozol™), {4-(5,6,7,8-Tetrahydro-imidazo-[1,5a]-pyridin-5-yl) benzonitril Monohydrochlorid (Fadrozol™) und Pyridoglutethimid (Rogletimid™).
Hinsichtlich der Bezeichnungen dieser Substanzen sowie deren Verfügbarkeit siehe beispielsweise "Rote Liste", Editio Cantor, Aulendorf (DE), (1985).

Solche Aromataseinhibitoren sind an sich bekannt, aber auf einem ganz anderen Gebiet, nämlich als systemisch eingesetzte Wirkstoffe zur medizinisch-therapeutischen Behandlung von Brustkrebs. In diesem Zusammenhang wird verwiesen auf die Übersichtsartikel von A.M.H. Brodi in: "J. Steorid Biochem. Molec. Biol.", Vol. 49, No. 4-6, pp. 281-287 (1994), sowie P. E. Goss und K.M.E.H. Gwyn in: "Journal of Clinical Oncology", Vol. 12, No. 11, pp. 2460-2470 (1994). Zur Bestimmung der Aromatase-Inhibition und der nachfolgenden Östrogenreduzierung wird auf die in den genannten Übersichtsartikeln angegebenen, weiteren Literaturnachweise verwiesen, s. beispielsweise A.M.H. Brodi et al. in: "J. Steroid Biochem. Molec. Biol.", Vol. 7, pp. 787-793 (1976), und D.A. Marsh et al. in: "J. Med. Chem.", Vol. 28, pp. 788-795 (1985).
Spezielle Azolderivate und deren aromatasehemmende und antimycotische Wirkung werden ferner beschrieben in der EP-A-0 575 210.

Es hat sich überraschend gezeigt, daß in Soja-Gycinen (INCI-Name nach dem Linné-System) Substanzen mit aromatasehemmenden Eigenschaften enthalten sind, und daß diese aus Soja-Glycinen stammenden Aromatase-Inhibitoren im Rahmen der vorliegenden Erfindung eingesetzt werden können. Diese aus Soja-Glycinen stammenden Aromatase-Inhibitoren können leicht erhalten werden durch Bereitstellung von "Glycine Soja" (Sojabohnenöl bzw. -extrakt oder Sojasterol) und anschließender Isolierung der Komponente mit aromatasehemmender Wirkung über gängige Trennmethoden, wie der Flüssigkeitschromatographie, insbesondere mittels der HPLC.

Es hat sich ferner herausgestellt, daß sich die Aromatase-Inhibitorwirkung der Soja-Glycine steigern läßt, wenn die Soja-Glycine oxidativ behandelt wird.
Die Darstellung dieser aus Soja-Glycinen stammenden, oxidierten Form erfolgt auf einfache Weise durch Oxidation der Soja-Glycine (Sojabohnenöl bzw. -extrakt oder Sojasterol) und anschließender Isolierung der Komponente mit aromatasehemmender Wirkung über gängige Trennmethoden, wie der Flüssigkeitschromatographie, insbesondere mittels der HPLC.
Die Oxidation kann auf enzymatische Weise, zum Beispiel gemäß der von Y.Fujimoto et al. in: "J. Am. Chem. Soc.", Vol. 104, pp. 4718-4720 (1982) beschriebenen Methode, oder auf chemischem Wege, wie zum Beispiel gemäß der von P.Welzel in: "Tetrahedron", Vol. 41, No. 20, pp. 4509-4517 (1985), beschriebenen Methode.

Als Beispiele für Substanzen der Anti-Östrogenklasse sind insbesondere die nicht-steroidalen Östrogen-Antagonisten Tamoxifen (Z-2-[4-(1,2-Diphenyl-1-butenyl)-phenoxy]-N,N-dimethylamin) und Aminoglutethimid (3-(4-Aminophenyl)-3-ethyl-2,6-piperidin-dion) sowie deren Analoga und Derivate, beispielsweise das 3-Hydroxytamoxifen, das 4-Hydroxytamoxifen sowie das 7 α-Alkyl-Sulfinyl-Tamoxifen-Analoge (ICI 182,780), zu erwähnen.
Hinsichtlich der Bezeichnungen dieser Substanzen sowie deren Verfügbarkeit siehe beispielsweise "Rote Liste", Editio Cantor, Aulendorf (DE), (1985).

Auch diese Anti-Östrogene sind bisher stets nur im Zusammenhang mit der systemisch therapeutischen Behandlung von Brustkrebs beschrieben worden.

Eine gesteigerte Effizienz bei der Straffung und/oder Verkleinerung von Fettzellen-haltigen Körperpartien ergibt sich aus der Wahl von erfindungsgemäß einzusetzenden Substanzen, die einen lipophilen Charakter aufweisen. Es hat sich gezeigt, daß lipophile Substanzen, insbesondere wenn sie in ein geeeignetes pharmakologisches Hautpermeations-Vehikelsystem eingebracht sind, leicht durch die Hautschichten sowie Zellmembranen passieren und somit schnell und effizient ihre oben erläuterte Wirkung entfalten können. Solche Substanzen bzw. die diese Substanzen enthaltenden Zusammensetzungen sind daher besonders gut für die topische Anwendung geeignet. Dies trifft insbesondere auf die steroidalen Aromatase-Inhibitoren zu, die von Natur aus durch das Steroid-Gerüst einen lipophilen Charakter besitzen und in der Regel eine gute bis sehr gute perkutane Resorptionsfähigkeit und Zellmembrangängigkeit aufweisen. Zudem können sich die lipophilen Substanzen leicht im Fettgewebe anreichern.
Substanzen, die von Natur aus eine erwünschte, hohe Lipophilie nicht aufweisen, können auf an sich bekannte Weise durch Modifizierung bzw. Derivatisierung mit lipohilen Gruppen lipophil gemacht werden, wobei allerdings darauf zu achten ist, daß die Hemmung der Bildung und/oder der Wirkung von Östrogenen nicht verlorengeht.

Falls in einzelnen Fällen die perkutane Resorption Probleme bereitet, oder falls eine gesteigerte perkutane Resorption erreicht werden soll, können in der verwendeten Zusammensetzung vorzugsweise zusätzlich Mittel zur Förderung der perkutanen Resorption eingesetzt werden. Solche Mittel zur Förderung der perkutanen Resorption sind bekannt.

Beispielsweise eignen sich hierfür Hyaluronidate, Dimethylsulfoxid (DMSO) und dergleichen.

Damit sich die Wirkmechanismen gegenseitig ergänzen und günstig beeinflussen können, wird gemäß einer bevorzugten Ausführungsform eine Zusammensetzung zur lokal topischen Applikation eingesetzt, welche ein oder mehrere Aromatase-Inhibitoren und ein oder mehrere Anti-Östrogene kombiniert umfaßt. Das bei der Kombination zu verwendende Mengenverhältnis ist dabei unkritisch und kann den jeweiligen Bedürfnissen angepaßt werden. So kann zum Beispiel jeweils die eine Substanzart oder die andere Substanzart überwiegen, je nachdem, welcher Wirkungsweg vorrangig angestrebt wird. Das gewichtsmäßige Mengenverhältnis vön Aromatase-Inhibitor zu Anti-Öströgen liegt beispielsweise in einem Bereich von 90/10 bis 10/90, insbesondere in einem Bereich von 60/40 bis 40/60.

Zur topischen Anwendung kann eine hierfür geeignete Formulierung des zu verwendenden Stoffs gewählt werden, z.B. eine Salbe, eine Creme, ein Gel, eine Emulsion (Lotio), ein Puder, ein Öl usw.. Zu diesem Zweck umfaßt die Zusammensetzung Zusatzstoffe, die für die entsprechende Formulierung als Salbe, Creme, Gel, Emulsion, Puder oder Öl usw. üblich sind. Beschriebene sowie handelsübliche, herkömmliche Hautpflegemittel sind in den jeweiligen Formulierungen zum Einsatz in der vorliegenden Erfindung bestens geeignet. Als übliche Zusatzstoffe für solche Formulierungen dienen beispielsweise pflanzliche Öle wie Mandelöl, Olivenöl, Pfirsichkernöl, Erdnußöl, Ricinusöl u. dergl., Pflanzenextrakte, etherische Öle, Vitaminöle, Fette und fettähnliche Stoffe, Lipoide, Phosphatide, Kohlenwasserstoffe wie Paraffine, Vaseline, Lanolin, Wachse u. dergl., Detergentien, weitere Hautwirkstoffe wie Lecithin, Wollfett alkohole, Carotin u. dergl., Hautnährstoffe, Parfums, kosmetische Stoffe, Alkohole, Glycerol, Glykole, Harnstoff, Talk, Konservierungsmittel, Sonnenschutzmittel, Farbstoffe wie Titanweiß und Zinkweiß, Antioxidantien usw.. Als Grundsubstanz dient im allgemeinen Wasser, so daß - üblicherweise unter Zusatz von Emulgatoren wie Fettalkoholsulfate, Alkaliseifen, Lecitine, Triethanolamin u. dergl. - eine O/W- oder W/O-Emulsion erhalten wird.

Sehr gut einsetzbar sind auch sogenannte transdermaltherapeutische Systeme (TTS) für die Haut, bei dem der Wirkstoff über ein klebefähiges Trägersystem, beispielsweise ein Pflaster, kontinuierlich über einen längeren Zeitraum in geeigneten Dosen auf die Haut appliziert werden kann.

Die Menge der Wirksubstanz zur Hemmung der Östrogenbildung bzw. -wirkung in solchen Formulierungen sind nicht kritisch und können auf den jeweiligen Anwendungsfall angepaßt werden. Geeignet ist beispielsweise ein Wirkstoffgehalt in der gesamten Zusammensetzung von 0,0001 bis 10 Gewichtsprozent (Gew.%), vorzugsweise 0,001 bis 5 Gew.% und insbesondere 0,3 bis 2 Gew.%.

Der Gehalt des ggf. einzusetzenden Resorptionsfördermittels hängt in erster Linie von der Art des Resorptionsfördermittels ab. Die jeweils herkömmlich eingesetzten Gehaltswerte sind dabei völlig geeignet. Hyaluronidate beispielsweise können in einer Konzentration von 0,01 bis 1 Gew.%, insbesondere 0,05 bis 0,2 Gew.% verwendet werden. Für DMSO ist ein weiterer Gehaltsbereich geeignet, beispielsweise 1 bis 25 Gew.%, insbesondere 5 bis 10 Gew.%.

Die weiteren, gegebenenfalls vorhandenen Zusatzstoffe können in den für die jeweiligen Formulierungen üblichen Mengen eingesetzt werden.

Zur Behandlung für die Straffung und/oder Verkleinerung von Fettzellen-haltigen Körperpartien braucht die beschriebene Substanz bzw. die diese Substanz enthaltende Zusammensetzung lediglich regelmäßig auf die zu behandelnden Körperpartien aufgetragen und leicht einmassiert zu werden (beispielsweise ein- bis zweimal täglich). Alternativ wird das transdermaltherapeutische System (TTS) auf den entsprechenden Hautbereich aufgebracht.

Der erfindungsgemäße Behandlungsweg ist auf beliebige Körperpartien äußerlich anwendbar, die gestrafft oder reduziert bzw. verkleinert werden sollen. Applikationsbereiche sind beispielsweise Brust-, Gesäß-, Oberarm- und Oberschenkelbereich. Aufgrund des besonderen Bedürfnisses an einer effizienten Möglichkeit zur Verkleinerung und/oder Sraffung der weiblichen Brust ist dies der bevorzugte Anwendungsbereich für die vorliegende Erfindung.

Die vorliegende Erfindung wird nachstehend anhand folgenden Beispiels beispielhaft erläutert.

### Beispiel

Folgende Bestandteile wurden zur Herstellung einer Creme zusammengemischt:

| | |
|---|---|
| Harnstoff | 10,0 g |
| Titanoxid | 15,0 g |
| Vaseline | 25,0 g |
| Isopropylpalmitat | 10,0 g |
| gehärtetes Erdnußöl | 10,0 g |
| Tween 80 | 5,0 g |
| 4-Hydroxy-Androstendion | 1,5 g |
| ger. Wasser ad. | 100,0 g |

Einer Probandin, 26 Jahre alt, die sowohl eine Erschlaffung beider Brüste über die Altersnorm hinaus als auch eine unerwünschte Vergrößerung des Busens aufwies, wurden 1 x täglich die obenstehende Creme lokal topisch appliziert. Die Creme enthielt einen Anteil von 1,5 Gew.-% 4-Hydroxy-Androstendion mit Aromatase-Hemmwirkung.

Bereits nach 6 Wochen zeigte sich eine deutliche Straffung der Haut über beiden Brüsten, die nach 10 Wochen ein Optimum erreichte.
Nach 12 Woche täglicher Anwendung war das Volumen beider Brüste um ca. 10% verkleinert. Die gewünschte Reduktion des. Busenvolumens um 30% wurde nach 24 Wochen erreicht. Bei täglicher Anwendung über ein weiteres Halbjahr blieben sowohl die Straffung der Haut als auch das reduzierte Busenvolumen erhalten.

## Patentansprüche

1. Verwendung einer Substanz, die. die Bildung und/oder die Wirkung von Östrogenen hemmt, oder einer diese Substanz enthaltenden Zusammensetzung, bei lokal topischer Applikation für kosmetische Zwecke zur Verkleinerung von Fettzellen-haltigen. Körperpartien.

2. Verwendung nach Anspruch 1, **dadurch gekennzeichnet, daß** die Substanz aus der aus Aromatase-Inhibitoren und Anti-Östrogenen bestehenden Gruppe ausgewählt ist.

3. Verwendung nach Anspruch 1, **dadurch gekennzeichnet, daß** die Substanz ein Aromatase-Inhibitor ist.

4. Verwendung nach Anspruch 1, **dadurch gekennzeichnet, daß** die Substanz aus Soja-Glycine stammt.

5. Verwendung nach Anspruch 4, **dadurch gekennzeichnet, daß** die Substanz aus Soja-Glycine oxidativ behandelt ist.

6. Verwendung nach einem der vorangehenden Ansprüche, **dadurch gekennzeichnet, daß** die Substanz einen lipophilen Charakter aufweist.

7. Verwendung nach einem der vorangehenden Ansprüche, **dadurch gekennzeichnet, daß** die Substanz in einer Zusammensetzung in einer Menge von 0,3 bis 1 Gew.-%, bezogen auf die Gesamtmenge der Zusammensetzung, enthalten ist.

8. Verwendung nach einem der vorangehenden Ansprüche, **dadurch gekennzeichnet, daß** die Zusammensetzung sowohl einen Aromatase-Inhibitor als auch ein Anti-Östrogen umfaßt.

9. Verwendung nach einem der vorangehenden Ansprüche, **dadurch gekennzeichnet, daß** die Substanz in einer Zusammensetzung, enthalten ist, die ferner Mittel zur Förderung der perkutanen. Resorption umfaßt.

10. Verwendung nach einem der vorangehenden Ansprüche, **dadurch gekennzeichnet, daß** die Substanz in einer Zusammensetzung enthalten ist, die als Salbe, Creme, Gel, Öl oder Emulsion bzw. Lotio formuliert ist.

11. Verwendung nach Anspruch 8, **dadurch gekennzeichnet, daß** die Zusammensetzung. Zusatzstoffe umfaßt, die für die entsprechende Formulierung als Salbe, Creme, Gel, Öl Emulsion bzw. Lotio üblich sind.

12. Verwendung einer Substanz, die die Bildung und/oder die Wirkung von Östrogenen hemmt, oder einer diese Substanz enthaltenden Zusammensetzung zur Herstellung eines Mittels zur Verkleinerung von Fettzellen-haltigen Körperpartien.

13. Verwendung nach Anspruch 12, **dadurch gekennzeichnet, daß** eine Substanz bzw. eine Zusammensetzung verwendet wird, wie sie in einem der Ansprüche 2 bis 11 definiert ist.

14. Verwendung einer Substanz, die die Bildung und/oder die Wirkung von Östrogenen hemmt, oder einer diese Substanz enthaltenden Zusammensetzung, bei lokal topischer Applikation für kosmetische Zwecke zur Straffung und/oder Verkleinerung des Brustbereichs.

15. Verwendung einer Substanz, die die Bildung und/oder die Wirkung von Östrogenen hemmt, oder einer diese Substanz enthaltenden Zusammensetzung zur Herstellung eines Mittels zur Straffung und/oder Verkleinerung des Brustbereichs.

16. Verwendung nach Anspruch 14 oder 15, **dadurch gekennzeichnet, daß** eine Substanz bzw. eine Zusammensetzung verwendet wird, wie sie in einem der Ansprüche 2 bis 11 definiert ist.

## Claims

1. Use of a substance which inhibits the formation and/or effect of oestrogens, or use of a composition containing this substance, for locally topical application for cosmetic purposes to reduce the size of body parts containing fat cells.

2. Use according to Claim 1, **characterized in that** the substance is chosen from the group consisting of aromatase-inhibitors and anti-oestrogens.

3. Use according to Claim 1, **characterized in that** the substance is an aromatase-inhibitor.

4. Use according to Claim 1, **characterized in that** the substance is derived from soyaglycine.

5. Use according to Claim 4, **characterized in that** the substance derived from soyaglycine is treated oxidatively.

6. Use according to any one of the preceding claims, **characterized in that** the substance is of a lipophile character.

7. Use according to any one of the preceding claims, **characterized in that** the substance is contained in a composition In a proportion of 0.3 to 1 %wt of the total quantity of the composition.

8. Use according to any one of the preceding claims, **characterized In that** the composition includes both en aromatase-inhibitor and an anti-oestrogen.

9. Use according to any one of the preceding claims, **characterized in that** the substance is contained in a composition which also includes agents for promoting percutaneous resorption.

10. Use according to any one of the preceding claims, **characterized in that** the substance is contained In a composition that is formulated as ointment, cream, gel, oil or emulsion or lotion,

11. Use according to Claim 6, **characterized In that** the composition includes usual additives for the corresponding formulation as ointment, cream, gel, oil, emulsion or lotion.

12. Use of a substance which inhibits the formation and/or effect of oestrogens, or use of a composition containing this substance, for the manufacture of a product for reducing the size of body parts containing fat cells.

13. Use according to Claim 12, **characterized in that** a substance or composition as defined in any one of Claims 2 to 11 is used.

14. Use of a substance which inhibits the formation and/or effect of oestrogens, or use of a composition containing this substance, for locally topical application for cosmetic purposes to firm, and/or reduce the size of, the breast region.

15. Use of a substance which inhibits the formation and/or effect of oestrogens, or use of a composition containing this substance, for the manufacture of a product for firming, and/or reducing the size of, the breast region.

16. Use according to Claim 14 or Claim 15, **characterized In that** a substance or composition as defined in any one of Claims 2 to 11 is used.

## Revendications

1. Utilisation d'une substance qui inhibe la formation et/ou l'action d'oestrogènes, ou d'une composition contenant cette substance, par application topique locale à des fins cosmétiques pour la réduction de la taille de parties du corps renfermant des cellules graisseuses.

2. Utilisation selon la revendication 1, **caractérisée en ce que** la substance est choisie dans le groupe constitué d'inhibiteurs d'aromatases et d'anti-oestrogènes.

3. Utilisation selon la revendication 1, **caractérisée en ce que** la substance est un inhibiteur d'aromatases,

4. Utilisation selon la revendication 1, **caractérisée en ce que** la substance est issue de la glycine de soja.

5. Utilisation selon la revendication 4, **caractérisée en ce que** la substance issue de la glycine de soja est traitée par oxydation.

6. Utilisation selon l'une des revendications précédentes, **caractérisée en ce que** la substance présente un caractère lipophile.

7. Utilisation selon l'une des revendications précédentes, **caractérisée en ce que** la substance est contenue dans une composition en une quantité comprise de 0,3 à 1% en poids par rapport la quantité totale de la composition.

8. Utilisation selon l'une des revendications précédentes, **caractérisée en ce que** la composition comprend non seulement un inhibiteur d'aromatases mais également un anti-oestrogène.

9. Utilisation selon l'une des revendications précédentes, **caractérisée en ce que** la substance est contenue dans une composition qui comprend en outre des moyens pour accélérer la résorption percutanée.

10. Utilisation selon l'une des revendications précédentes, **caractérisée en ce que** la substance est contenue dans une composition qui est sous la forme d'une pommade, d'une crème, d'un gel, d'une huile ou d'une émulsion, éventuellement d'une lotion.

11. Utilisation selon la revendication 8, **caractérisée en ce que** la composition comprend des additifs, qui sont usuels pour les formulations correspondantes telles qu'une pommade, une crème, un gel, une émulsion, éventuellement une lotion.

12. Utilisation d'une substance qui inhibe la formation et/ou l'action d'oestrogènes, ou d'une composition contenant cette substance, pour la fabrication d'un moyen à utiliser pour réduire la taille de parties du corps renfermant des cellules graisseuses.

13. Utilisation selon la revendication 12, **caractérisée en ce que** l'on utilise une substance, éventuellement une composition, telle que définie dans l'une des revendications 2 à 11.

14. Utilisation d'une substance qui inhibe la formation et/ou l'action d'oestrogènes, ou d'une composition contenant cette substance, par application topique locale à des fins cosmétiques pour le raffermissement et/ou la réduction de la taille de la région de la poitrine.

15. Utilisation d'une substance, qui inhibe la formation et/ou l'action d'oestrogènes, ou d'une composition contenant cette substance, pour la fabrication d'un moyen pour le raffermissement et/ou la réduction de la taille de la région de la poitrine.

16. Utilisation selon la revendication 14 ou 15, **caractérisée en ce que** l'on utilise une substance, éventuellement une composition, telle que définie dans l'une des revendications 2 à 11.
